(19) Europäisches Patentamt
European Patent Office
Office européen des brevets

(11) Publication number: **0 280 534 B1**

(12) # EUROPEAN PATENT SPECIFICATION

(45) Date of publication of patent specification: **21.04.93**

(51) Int. Cl.5: **A61K 37/26**, C07K 99/26, C07K 7/40

(21) Application number: **88301590.1**

(22) Date of filing: **24.02.88**

(54) **Novel insulin derivatives.**

(30) Priority: **25.02.87 DK 948/87**
**10.07.87 DK 3569/87**
**16.10.87 DK 5400/87**

(43) Date of publication of application:
**31.08.88 Bulletin 88/35**

(45) Publication of the grant of the patent:
**21.04.93 Bulletin 93/16**

(84) Designated Contracting States:
**AT BE CH DE ES FR GB GR IT LI LU NL SE**

(56) References cited:
**EP-A- 0 060 141**
**EP-A- 0 214 826**
**US-A- 3 883 500**
**EP-A- 0 132 769**
**GB-A- 2 104 382**
**US-A- 3 884 897**

(73) Proprietor: **NOVO NORDISK A/S**
**Novo Allé**
**DK-2880 Bagsvaerd(DK)**

(72) Inventor: **Langkjaer, Liselotte**
**22C Kroyersvej**
**DK-2930 Klampenborg(DK)**
Inventor: **Norris, Kjeld**
**34 Ahlmanns Allé**
**DK-2900 Hellerup(DK)**
Inventor: **Markussen, Jan**
**7 Kikudbakken**
**DK-2730 Herlev(DK)**
Inventor: **Jorgensen, Klavs Holger**
**47 Askevaenget**
**DK-2830 Virum(DK)**

(74) Representative: **Brown, John David et al**
**FORRESTER & BOEHMERT Franz-Joseph-Strasse 38**
**W-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

The present invention relates to novel insulin derivatives having improved properties, to methods for their preparation and to preparations containing such novel insulin derivatives.

In the treatment of diabetes mellitus, many varieties of insulin preparations have been suggested and used. Even though improved insulin preparations have steadily been invented during the insulin era, there is still a need for insulin preparations with improved properties.

Acidic solutions of insulin have been used earlier, both as short-acting preparations and together with protamine and/or zinc as long-acting preparations. However, under ordinary circumstances the chemical stability of insulin at pH values below 4.5 is low, as formation of desamidoinsulins (Sundby, F., J.Biol.Chem. 237 (1962), 3406 - 3411) and covalent dimers (Steiner et al., Diabetes 17 (1968), 725 - 736) takes place. In the pH range 4.5 - 6.5, insulin precipitates. Hence, in order to obtain soluble short-acting insulin preparations (by the addition of blood-flow enhancing agents) and long-acting insulin preparations (by the addition of protamine and/or zinc) an insulin stable at a low pH would be desirable.

One object of this invention is to provide insulin derivatives with improved properties.

A second object of this invention is to provide solutions of insulin derivatives having an improved stability.

A third object of this invention is to provide preparations of insulin derivatives with low or with no immunogenic activity.

A fourth object of this invention is to provide insulin preparations which are soluble at pH values from 2.0 to 8.0, preferably from 2.0 to 4.5 and from 6.5 to 8.0.

A fifth object of this invention is to provide solutions of insulin derivatives having an improved stability at pH values of 3-4.

A sixth object of this invention is to provide long-acting solutions of insulin derivatives.

The present invention relates to human insulin, porcine insulin, rabbit insulin and des(B30) human insulin wherein the A21 amino acid has been substituted by Ala, Gln, Glu, Gly, His, Ile, Leu, Met, Phe, Ser, Thr, Trp, Tyr, Val or hSer.

Such compounds can be designated by the general formula I

```
INSUL-A21
    |                                   (I)
   B30
```

wherein INSUL represents des(A21),des(B30) human insulin and A21 represents one of the amino acids Ala, Gln, Glu, Gly, His, Ile, Leu, Met, Phe, Ser, Thr, Trp, Tyr, Val or hSer connected to $Cys^{A20}$ in INSUL, and B30 represents hydrogen or one of the amino acids Ser, Ala or Thr connected to $Lys^{B29}$ in INSUL.

It is known that during the acidic ethanol extraction of mammalian insulins many dimers are formed (Steiner) and, furthermore, monodesamidoinsulins are formed under acid conditions (Sundby).

It has now, surprisingly, been found that the formation of such undesired dimers is substantially reduced or almost eliminated when the insulin compound used is one of the above insulin derivatives wherein $Asn^{A21}$ has been exchanged with one of the above-mentioned amino acids. This substitution also eliminates the formation of monodesamido insulins.

The novel insulin derivatives have the following advantages:

1) The formation of the immunogenic dimers, i.e. covalently linked insulin molecules linked either through the two A-chains, (AA) dimer, or through one A-chain and one B-chain, (AB) dimer, (Helbig, H.J., Deutsche Wollforschungsinstitut, dissertation, 1976) is substantially eliminated (a chromatographic fraction of crude porcine insulin, the b-component, containing the dimers was shown to be immunogenic in rabbits (Schlichtkrull et al., Horm.Metab.Res. Suppl. 5 (1974), 134 - 143)).

2) The stability of the novel insulin derivatives is so high that it will probably be possible to store preparations containing these novel insulin derivatives at room temperature for a long period of time. This will be a major advantage for the patient.

3) It will be possible to prepare dissolved preparations containing the novel insulin derivatives at pH values from 2 to 8, preferably in the range of from 2.5 to 8 more preferred from 2.5 to 4.5 and from 6.5 to 8.0.

4) it will be possible to prepare preparations containing the novel insulin derivatives which, at pH values of about 3, have a substantially improved chemical stability.

5) In the pH range of 3-4, which is inappropriate for mammalian insulin because of chemical instability, useful solutions of insulin derivatives can be made in the presence of magnesium ions in concentrations of 0.005 M to 0.5 M, preferably with insulin derivatives of formula I wherein A21 is different from Gln.

6) It will be possible to prepare soluble, rapidly acting preparations containing the novel insulin derivatives by the addition of compounds which enhance the absorption.

7) It will be possible to prepare soluble, retarded preparations containing the novel insulin derivatives by the addition of zinc and/or protamine to acid solutions, i.e. solutions having a pH value in the range from 2.5 to 4.

8) It will be possible to prepare preparations containing the novel insulin derivatives having different profiles.

Compounds of formula I may be prepared by a transpeptidation reaction in which a biosynthetic precursor compound having the general formula II

```
INSUL-A21
    |                                              (II)
    X
```

wherein A21 is as defined above, and X is a bond, an amino acid residue or a peptide residue bridging the carboxyl group of $Lys^{B29}$ to the amino group of $Gly^{A1}$, is reacted with an amino compound of the general formula III

Z-OR (III)

wherein Z is Thr, Ala or Ser wherein any hydroxy group may be protected, and R is a carboxy protecting group (e.g. methyl or tert-butyl), using trypsin or a trypsin-like enzyme as a catalyst in a mixture of water and organic solvents analogously as described in US patent specification No. 4,343,898, whereafter the carboxy protecting group and any hydroxy protecting group is removed. X may for example by a moiety of the formula IV

$-(Q_q\text{-}K)_r-$ (IV)

wherein Q is a peptide chain and q amino acids, q is an integer from 0 to 33, K is Lys or Arg, and r is zero or one.

Compounds of the formula II may be prepared by a method similar to the method described in European patent application Nos. 163,529 and 214.826. By this method a DNA-sequence encoding a compound with the formula II is inserted into a suitable expression vector which, when transferred to a suitable yeast strain, is capable of expressing the desired compound with correctly positioning disulphide bridges. The product expressed is then isolated from the cells or the culture broth depending on whether it is secreted from the cells or not.

At neutral pH, compounds of formula I have the same charge as human insulin. In solution, compounds of formula I may be present as hexamers.

Examples of specific preferred compounds according to this invention are the following: $Gly^{A21}$ human insulin, $Ala^{A21}$ human insulin, $Ser^{A21}$ human insulin, $Thr^{A21}$ human insulin, $hSer^{A21}$ human insulin, $Gly^{A21}$ porcine insulin, $Ala^{A21}$ porcine insulin, $Ser^{A21}$ porcine insulin and $Thr^{A21}$ porcine insulin.

Insulin preparations of this invention can be prepared by dissolving a compound of formula I in an aqueous medium at slightly acidic conditions, for example, in a concentration of from 240 to 600 nmole/ml.

The aqueous medium can be made isotonic by the addition of sodium chloride, sodium acetate or glycerol.

If a protracted preparation is required the above mentioned isotonic agents can in part or completely be replaced by a zinc salt or a mixture of zinc salts at a concentration of up to 5 $\mu$g $Zn^{2+}$ per nmol of compound of formula I.

Further, it has been found that many magnesium salts have a solubilising effect on insulin at pH values of from 4 to 6.2 and an enhancing effect on the absorption of insulin.
Various mixtures of magnesium salts have the same effect. It is, therefore, concluded that the presence of magnesium ions at certain concentrations is a critical parameter for the solubility of insulin at pH values of 4 to 6.2 and for the rate of absorption. The range of applicable magnesium ion concentration is from 0.005 M

to 0.5M, preferably above 0.05 M. The upper limit is somewhat arbitrary being chosen from the assumption that in some cases (e.g. for intraperitoneal infusion) some overstepping of isotonicity may be acceptable. According to a preferred embodiment of this invention the preparations contain magnesium ions in a concentration of from 0.08 M to 0.3 M.

It has furthermore been found that protracted - or further protracted - preparations of the insulin derivatives of this invention are obtained when protamine is added to the above mentioned preparations, i.e. the preparations containing no zinc ions and no magnesium ions, the preparations containing zinc ions and the preparations containing magnesium ions. The amount of protamine to be used is from 5% to 50%, preferably from 8% to 40%, more preferred from 10% to 30% on the basis of insulin (weight/weight).

Insulin preparations with enhanced absorption properties can also be obtained by the addition of arginine or lysine to an aqueous solution of the insulin. The preferred concentration of these amino acids is from 0.01 M to 0.2 M.

The insulin preparations may further contain buffers such as acetate and citrate and preservatives such as phenol, m-cresol and methyl paraben. The pH of the solution is adjusted to the desired value and the insulin preparation is made sterile by sterile filtration.

Insulin solutions of this invention having a pH value in the range 3 - 6.2 may also be particularly useful for the purpose of infusion by means of pumps, because of a lack of insulin precipitation caused by carbon dioxide diffusion through catheters. Such precipitation has been observed occasionally with neutral infusion solutions, and is believed to be attributable to the lowering of the pH value caused by carbon dioxide.

The abbreviations used herein for the amino acid residues are those state in J.Biol.Chem. 243 (1968), 3558. The amino acids stated herein are in L configuration. Within the context of this invention the term insulin when used in a plural or generic sense is intended to encompass both naturally occuring insulins and insulin derivatives. $Gly^{A21}$ human insulin is human insulin wherein $Asn^{A21}$ has been exchanged by Gly and similarly for similar names.

The insulin preparations of this invention can be used in the treatment of diabetes. It is recommended that the dosage of the insulin preparations of this invention be selected by a physician similarly to the selection of the dosage of known insulin preparations for injection.

Any novel feature or combination of features described herein is considered essential to this invention.

Example 1

Preparation of $Gly^{A21}$ Human Insulin

$Gly^{A21}$ human insulin was prepared by transpeptidation of a compound which according to formula II can be formulated as

```
INSUL-Gly^A21
     |                                   (V)
     Ala-Ala-Lys-
```

wherein the terminal Ala of the bridging peptide is linked to the carboxyl group of $Lys^{B29}$ and Lys is linked to the amino group of $Gly^{A1}$, with Thr-OMe (L-threonine methylester) followed by hydrolysis of the ester group with aqueous sodium hydroxide. Thus 100 mg of the compound of formula V was dissolved in 0.5 ml of 10 M acetic acid and 1 ml of 2 M Thr-OMe in N,N-dimethylacetamide was added. The mixture was cooled to 12° C. 10 mg of trypsin dissolved in 0.2 ml of 0.05 M calcium acetate was added. After 48 hours at 12° C the proteins were precipitated by addition of 20 ml of acetone. The conversion of the starting material into $Gly^{A21}$-$(Thr\text{-}OMe)^{B30}$ human insulin was 88% by HPLC.

250 mg of $Gly^{A21}$-$(Thr\text{-}OMe)^{B30}$ human insulin was suspended in 25 ml of water and dissolved by the addition of 1 N sodium hydroxide solution to a pH value of 10.0. The pH value is kept constant at 10.0 for 24 hours at 25° C. The insulin derivative formed was crystallized by the addition of 2 g of sodium chloride, 350 mg of sodium acetate trihydrate and 2.5 mg of zinc acetate dihydrate followed by the addition of 1 N hydrochloric acid to obtain a pH value of 5.52. After 24 hours at 4° C the crystallized material was isolated by centrifugation washed with 3 ml of water, isolated by centrifugation, and dried *in vacuo*. Yield: 210 mg of $Gly^{A21}$ human insulin.

The compound of formula V was prepared by a method analogous to example 2 of European patent application No. 214.826.

Example 2

Preparation of Injectable Solution of Compounds of Formula I

15 µmol of $Gly^{A21}$ human insulin containing 0.5% of zinc are dissolved in water (5 ml) containing hydrochloric acid (80 µl of 1 N) followed by the addition of an aqueous solution (10 ml) containing phenol (65 mg) and glycerol (400 mg). The pH value of the solution is adjusted to 3.0 by means of a sodium hydroxide solution and the total volume is adjusted to 25 ml with water. The resulting solution is sterilized by filtration and subsequently transferred aseptically to vials (5 ml).

Example 3

Soluble Preparation of $Gly^{A21}$ Human Insulin with Protracted Action

15 µmol of $Gly^{A21}$ human insulin (zinc free) are dissolved in water (5 ml). To this solution is added hydrochloric acid (80 µl of 1 N) and zinc chloride (100 µl of 0.6 M) followed by the addition of an aqueous solution (15 ml) containing protamine sulphate (37 mg), m-cresol (50 mg) and sodium chloride (200 mg). The pH is adjusted to 3.5 with sodium hydroxide solution and the total volume is adjusted to 25 ml with water. Finally, the solution is sterilized by filtration and transferred aseptically to sterile vials.

The absorption profile after subcutaneous injection in pigs was found comparable to that of the well known insulin suspension Protaphane®HM 100 IU/ml.

Example 4

Soluble Preparation of $Gly^{A21}$ Human Insulin with Fast Action

15 µmol of $Gly^{A21}$ human insulin (zinc free) are dissolved in water (10 ml). To this solution is added hydrochloric acid (40 µl of 1 N) and magnesium chloride (2.6 ml of 1 M) followed by the addition of an aqueous solution of benzyl alcohol (8 ml of 0.3 M). The pH is adjusted to 5.7 with sodium hydroxide solution and the total volume is adjusted to 25 ml with water. Finally the solution is sterilized by filtration and transferred aseptically to sterile vials.

Example 5

Chemical Stability of $Gly^{A21}$ Human Insulin in Preparations

Three preparations containing 0.24 mM of $Gly^{A21}$ human insulin (zinc free), 0.26% (w/v) of phenol and 1.6 % (w/v) of glycerol were prepared and their pH value adjusted to 3.0, 4.0, and 5.0, respectively.

Samples were analyzed after storage at 45 °C for two weeks using human insulin preparations of the same composition as reference.

Table 1 shows the content of insulin dimerization and polymerization products as determined by HPSEC (High Performance Size Exclusion Chromatography).

Table 2 shows the content of insulin deamidation products determined by DISC PAGE (Poly Acrylamide Gel Electrophoresis).

Table 1

| pH of Preparation | Human Insulin | $Gly^{A21}$ Human Insulin |
|---|---|---|
| 3.0 | 4.9% | 0.31% |
| 4.0 | 41.6% | 1.0% |
| 5.0 | 16.1% | 2.8% |
| Dry Insulin | 0.29% | 0.05% |

Table 2

| pH of Preparation | Human Insulin | $Gly^{A21}$ Human Insulin |
|---|---|---|
| 3.0 | 90% | 2% |
| 4.0 | 40% | 3% |
| 5.0 | 3% | 4% |
| Dry Insulin | 0.5% | 0.5% |

Example 6

Biological Potency of $Gly^{A21}$ Human Insulin

Investigation according to the British Pharmacopeia, 1980 edition, of the potency of $Gly^{A21}$ human insulin showed that this was approximately 85% of that of human insulin. Within the dose range relevant for therapeutic purposes no toxic manifestations were observed.

Example 7

Soluble Preparation of $Gly^{A21}$ Human Insulin with Further Protracted Action

15 µmol of $Gly^{A21}$ human insulin (zinc free) are dissolved in water (5 ml). To this solution is added hydrochloric acid (80 µl of 1 N) and zinc chloride (100 µl of 0.6 M) followed by the addition of an aqueous

solution (15 ml) containing protamine sulphate (37 mg), m-cresol (50 mg) and magnesium chloride (200 mg). The pH is adjusted to 3.5 and the total volume is adjusted to 25 ml with water. Finally, the solution is sterilized by filtration and transferred aseptically to sterile vials.

The absorption of this preparation after subcutaneous injection in pigs was found to be substantially slower than that of the well known insulin suspension Protaphane®HM 100 IU/ml.

The features disclosed in the foregoing description and in the following claims may, both separately and in any combination thereof, by material for realising the invention in diverse forms thereof.

## Claims

### Claims for the following Contracting States : AT, BE, CH, DE, FR, GB, IT, LI, LU, NL, SE

**1.** Insulin derivatives of the general formula I

```
INSUL-A21
       |                    (I)
      B30
```

wherein INSUL represents des(A21), des(B30) human insulin, characterized in that A21 represents one of the amino acids Ala, Gln, Glu, Gly, His, Ile, Leu, Met, Phe, Ser, Thr, Trp, Tyr, Val or hSer connected to $Cys^{A20}$ in INSUL, and B30 represents hydrogen or one of the amino acids Ser, Ala or Thr connected to $Lys^{B29}$ in INSUL, and preferably A21 is different from Phe.

**2.** Insulin derivatives according to Claim 1, wherein A21 represents Gly, Ala, Ser, Thr, or hSer, and B30 represents Ala or Thr.

**3.** Preparation characterized in that it contains a compound of formula I stated in Claim 1 or 2 above with the definitions stated therein.

**4.** Preparation according to Claim 3, characterized in that it is soluble.

**5.** Preparation according to Claim 4, characterized in that it contains a compound which enhances the absorption.

**6.** Preparation according to claim 5, characterized in that said compound is a magnesium salt.

**7.** Preparation according to claim 6, characterized in that it is a solution with a pH value in the range of 3-4 and that it contains magnesium ions in a concentration of 0.005 M to 0.5 M which preparation preferably contains a compound of formula I wherein A21 is different from Gln.

**8.** Preparation according to claim 5, characterized in that said compound is arginine or lysine.

**9.** Preparation according to Claim 3, 4, 6, 7 or 8 characterized in that it contains zinc ions and/or protamine.

**10.** Preparation according to any one of Claims 3 to 9, characterized in that it has a pH value in the range of from 2.0 to 8, preferably from 2.5 to 8.

**11.** Preparation according to any one of Claims 3 to 9, characterized in that it has a pH value in the range of from 2.5 to 4.5 or from 6.5 to 8.0.

**12.** Method for the preparation of insulin derivatives according to claim 1, wherein a biosynthetic precursor compound having the general formula II

INSUL-A21
|
X (II)

wherein A21 is as defined in claim 1, and X is a bond, an amino acid residue or a peptide residue bridging the carboxyl group of $Lys^{B29}$ to the amino group of $Gly^{A1}$, is reacted with an amino compound of the general formula III

Z-OR (III)

wherein Z is Thr, Ala or Ser wherein any hydroxy group may be protected, and R is a carboxy protecting group, using trypsin or a trypsin-like enzyme as a catalyst in a mixture of water and organic solvents whereafter the carboxy protecting group and any hydroxy protecting group is removed.

**13.** Method according to claim 12, wherein X is a moiety of the formula IV

$-(Q_q-K)_r-$ (IV)

wherein Q is a peptide chain with q amino acids, q is an integer from 0 to 33, K is Lys or Arg, and r is zero or one.

**Claims for the following Contracting States : ES, GR**

**1.** A method for the preparation of insulin derivatives of the general formula I

INSUL-A21
|
B30 (I)

wherein INSUL represents des(A21), des(B30) human insulin, and A21 represents one of the amino acids Ala, Gln, Glu, Gly, His, Ile, Leu, Met, Phe, Ser, Thr, Trp, Tyr, Val or hSer connected to $Cys^{A20}$ in INSUL, and B30 represents hydrogen or one of the amino acids Ser, Ala or Thr connected to $Lys^{B29}$ in INSUL, and preferably A21 is different from Phe, wherein a biosynthetic precursor compound having the general formula II

INSUL-A21
|
X (II)

wherein A21 is as defined above, and X is a bond, an amino acid residue or a peptide residue bridging the carboxyl group of $Lys^{B29}$ to the amino group of $Gly^{A1}$, is reacted with an amino compound of the general formula III

Z-OR (III)

wherein Z is Thr, Ala or Ser wherein any hydroxy group may be protected, and R is a carboxy protecting group, using trypsin or a trypsin-like enzyme as a catalyst in a mixture of water and organic solvents, whereafter the carboxy protecting group and any hydroxy protecting group is removed.

2. Method according to Claim 1, wherein X is a moiety of the formula IV

$-(Q_q\text{-}K)_r-$ (IV)

wherein Q is a peptide chain with q amino acids, q is an integer from 0 to 33, K is Lys or Arg, and r is zero or one.

3. A method according to Claim 1 or 2, wherein A21 represents Gly, Ala, Ser, Thr or HSer, and B30 represents Ala or Thr.

4. Use of an insulin derivative of the general formula I

```
INSUL-A21
   |                                   (I)
   B30
```

wherein INSUL represents des(A21), des(B30) human insulin, and A21 represents one of the amino acids Ala, Gln, Glu, Gly, His, Ile, Leu, Met, Phe, Ser, Thr, Trp, Tyr, Val or hSer connected to $Cys^{A20}$ in INSUL, and B30 represents hydrogen or one of the amino acids Ser, Ala or Thr connected to $Lys^{B29}$ in INSUL, and preferably A21 is different from Phe in a method of manufacturing a preparation for the treatment of diabetes mellitus.

5. Use according to Claim 4, wherein the preparation to be manufactured is soluble.

6. Use according to Claim 5, wherein the preparation to be manufactured contains a compound which enhances the absorption.

7. Use according to Claim 6, wherein said compound is a magnesium salt.

8. Use according to Claim 7, wherein the preparation to be manufactured is a solution with a pH value in the range of 3-4 and contains magnesium ions in a concentration of 0.005 M to 0.5 M, which preparation preferably contains a compound of formula I wherein A21 is different from Gln.

9. Use according to Claim 6, wherein said compound is arginine or lysine.

10. Use according to Claim 4, 5, 7, 8 or 9, wherein the preparation to be manufactured contains zinc ions and/or protamine.

11. Use according to any one of Claims 4 to 10, wherein the preparation to be manufactured has a pH value in the range of from 2.0 to 8, preferably from 2.5 to 8.

12. Use according to any one of Claims 4 to 10, wherein the preparation to be manufactured has a pH value in the range of from 2.5 to 4.5 or from 6.5 to 8.0.

**Patentansprüche**
**Patentansprüche für folgende Vertragsstaaten : AT, BE, CH, DE, FR, GB, IT, LI, LU, NL, SE**

1. Insulinderivate der allgemeinen Formel I

```
INSUL-A21
   |
   B30                                 (I)
```

wobei INSUL des(A21) des(B30)-Humaninsulin darstellt, dadurch gekennzeichnet, daß A21 eine der Aminosäuren Ala, Gln, Glu, Gly, His, Ile, Leu, Met, Phe, Ser, Thr, Trp, Tyr, Val oder hSer darstellt, die an das $Cys^{A20}$ in INSUL gebunden sind, und B30 Wasserstoff oder eine der Aminosäuren Ser, Ala oder Thr darstellt, die an das $Lys^{B29}$ in INSUL gebunden sind, und bevorzugt ist A21 von Phe verschieden.

2. Insulinderivat nach Anspruch 1, wobei A21 Gly, Ala, Ser, Thr oder hSer darstellt und B30 Ala oder Thr darstellt.

3. Präparat, dadurch gekennzeichnet, daß es eine Verbindung der Formel I, die oben in den Ansprüchen 1 oder 2 mit den darin angegebenen Definitionen angegeben ist, enthält.

4. Präparat nach Anspruch 3, dadurch gekennzeichnet, daß es löslich ist.

5. Präparat nach Anspruch 4, dadurch gekennzeichnet, daß es eine Verbindung enthält, die die Absorption verbessert.

6. Präparat nach Anspruch 5, dadurch gekennzeichnet, daß die Verbindung ein Magnesiumsalz ist.

7. Präparat nach Anspruch 6, dadurch gekennzeichnet, daß es eine Lösung mit einem pH-Wert im Bereich von 3 bis 4 ist und daß es Magnesiumionen in einer Konzentration von 0,005 M bis 0,5 M enthält, wobei das Präparat bevorzugt eine Verbindung der Formel I enthält, wobei A21 von Gln verschieden ist.

8. Präparat nach Anspruch 5, dadurch gekennzeichnet, daß die Verbindung Arginin oder Lysin ist.

9. Präparat nach Anspruch 3, 4, 6, 7 oder 8, dadurch gekennzeichnet, daß es Zinkionen und/oder Protamin enthält.

10. Präparat nach einem der Ansprüche 3 bis 9, dadurch gekennzeichnet, daß es einen pH-Wert in dem Bereich von 2,0 bis 8, bevorzugt von 2,5 bis 8, hat.

11. Präparat nach einem der Ansprüche 3 bis 9, dadurch gekennzeichnet, daß es einen pH-Wert in dem Bereich von 2,5 bis 4,5 oder von 6,5 bis 8,0 hat.

12. Verfahren zum Herstellen von Insulinderivaten nach Anspruch 1, bei dem eine biosynthetische Präkursor-Verbindung mit der allgemeinen Formel II

```
INSUL-A21
     |
     X                                   (II),
```

wobei A21 wie in Anspruch 1 definiert ist und X eine Bindung, ein Aminosäurerest oder ein Peptidrest ist, die/der die Carboxylgruppe von $Lys^{B29}$ zu der Aminogruppe von $Gly^{A1}$ überbrückt, mit einer Aminoverbindung der allgemeinen Formel III

Z-OR (III)

reagiert wird, wobei Z Thr, Ala oder Ser ist, wobei jegliche Hydroxygruppe geschützt werden kann, und R eine Carboxy-schützende Gruppe ist, welche Trypsin oder ein Trypsin ähnliches Enzym als Katalysator in einer Mischung aus Wasser und organischen Lösemitteln verwendet, wonach die Carboxy-schützende Gruppe und jegliche Hydroxy-schützende Gruppe entfernt wird.

13. Verfahren nach Anspruch 12, wobei X ein Anteil der Formel IV

$-(Q_q\text{-}K)_r-$ (IV)

ist, wobei Q eine Peptidkette mit q Aminosäuren ist, q eine ganze Zahl von 0 bis 33 ist, K Lys oder Arg ist und r Null oder Eins ist.

**Patentansprüche für folgende Vertragsstaaten : ES, GR**

1. Verfahren zum Herstellen von Insulinderivaten der allgemeinen Formel I

```
INSUL-A21
    |                    (I),
   B30
```

wobei INSUL des(A21), des(B30)-Humaninsulin darstellt und A21 eine der Aminosäuren Ala, Gln, Glu, Gly, His, Ile, Leu, Met, Phe, Ser, Thr, Trp, Tyr, Val oder hSer darstellt, die an $Cys^{A20}$ in INSUL gebunden sind, und B30 Wasserstoff oder eine der Aminosäuren Ser, Ala oder Thr darstellt, die an $Lys^{B29}$ in INSUL gebunden sind, und bevorzugt ist A21 von Phe verschieden, bei dem eine biosynthetische Präkursor-Verbindung mit der allgemeinen Formel II

```
INSUL-A21
    |                    (II),
    X
```

wobei A21 wie oben definiert ist und X eine Bindung, ein Aminosäurerest oder ein Peptidrest ist, der/die Carboxylgruppe von $Lys^{B29}$ zu der Aminogruppe von $Gly^{A1}$ überbrückt, mit einer Aminoverbindung der allgemeinen Formel III

Z-OR (III)

reagiert wird, wobei Z Thr, Ala oder Ser ist, wobei jegliche Hydroxygruppe geschützt werden kann, und R eine Carboxy-schützende Gruppe ist, wobei Trypsin oder ein Trypsin ähnliches Enzym als Katalysator in einer Mischung aus Wasser und organischen Lösemitteln verwendet wird, wonach die Carboxyschützende Gruppe und jegliche Hydroxy-schützende Gruppe entfernt wird.

2. Verfahren nach Anspruch 1, bei dem X ein Anteil der Formel IV

$-(Q_q-K)_r-$ (IV)

ist, wobei Q eine Peptidkette mit q Aminosäuren ist, wobei q eine ganze Zahl von 0 bis 33 ist, K Lys oder Arg ist und r Null oder Eins ist.

3. Verfahren nach Anspruch 1 oder 2, bei dem A21 Gly, Ala, Ser, Thr oder hSer darstellt und B30 Ala oder Thr darstellt.

4. Verwendung eines Insulinderivates der allgemeinen Formel I

```
INSUL-A21
    |                    (I),
   B30
```

wobei INSUL des(A21), des(B30)-Humaninsulin darstellt und A21 eine der Aminosäuren Ala, Gln, Glu, Gly, His, Ile, Leu, Met, Phe, Ser, Thr, Trp, Tyr, Val oder hSer darstellt, die an $Cys^{A20}$ in INSUL gebunden sind, und B30 Wasserstoff oder eine der Aminosäuren Ser, Ala oder Thr darstellt, die an $Lys^{B29}$ in INSUL gebunden sind, und bevorzugt ist A21 von Phe verschieden, in einem Verfahren zum Herstellen eines Präparates für die Behandlung von Diabetes mellitus.

**5.** Verwendung nach Anspruch 4, wobei das herzustellende Präparat löslich ist.

**6.** Verwendung nach Anspruch 5, wobei das herzustellende Präparat eine Verbindung enthält, die die Absorption verbessert.

**7.** Verwendung nach Anspruch 6, wobei die Verbindung ein Magnesiumsalz ist.

**8.** Verwendung nach Anspruch 7, wobei das herzustellende Präparat eine Lösung mit einem pH-Wert in dem Bereich von 3 bis 4 ist und Magnesiumionen in einer Konzentration von 0,005 M bis 0,5 M enthält, wobei das Präparat bevorzugt eine Verbindung der Formel I enthält, wobei A21 von Gln verschieden ist.

**9.** Verwendung nach Anspruch 6, wobei die Verbindung Arginin oder Lysin ist.

**10.** Verwendung nach Anspruch 4, 5, 7, 8 oder 9, wobei das herzustellende Präparat Zinkionen und/oder Protamin enthält.

**11.** Verwendung nach einem der Ansprüche 4 bis 10, wobei das herzustellende Präparat einen pH-Wert in dem Bereich von 2,0 bis 8, bevorzugt von 2,5 bis 8 hat.

**12.** Verwendung nach einem der Ansprüche 4 bis 10, wobei das herzustellende Präparat einen pH-Wert im Bereich von 2,5 bis 4,5 oder von 6,5 bis 8,0 hat.

**Revendications**

**Revendications pour les Etats contractants suivants : AT, BE, CH, DE, FR, GB, IT, LI, LU, NL, SE**

**1.** Dérivés de l'insuline de la formule générale I

INSUL-A21
|
B30 (I)

dans laquelle INSUL représente l'insuline humaine des(A21), des(B30), caractérisés en ce qu'A21 représente l'un des acides aminés Ala, Gln, Glu, Gly, His, Ile, Leu, Met, Phe, Ser, Thr, Trp, Tyr, Val ou hSer relié à $Cys^{A20}$ dans INSUL et B30 représente l'hydrogène ou l'un des acides aminés Ser, Ala ou Thr relié à $Lys^{B29}$ dans INSUL, et de préférence A21 est différent de Phe.

**2.** Dérivés de l'insuline selon la revendication 1, dans lesquels A21 représente Gly, Ala, Ser, Thr ou hSer et B30 représente Ala ou Thr.

**3.** Préparation caractérisée en ce qu'elle contient un composé de formule I désigné dans la revendication 1 ou 2 ci-dessus avec les définitions énoncées dans celle-ci.

**4.** Préparation selon la revendication 3, caractérisée en ce qu'elle est soluble.

**5.** Préparation selon la revendication 4, caractérisée en ce qu'elle contient un composé qui favorise l'absorption.

**6.** Préparation selon la revendication 5, caractérisée en ce que le composé est un sel de magnésium.

**7.** Préparation selon la revendication 6, caractérisée en ce qu'il s'agit d'une solution ayant une valeur de pH dans la plage de 3-4 et qui contient des ions magnésium dans une concentration de 0,005 M à 0,5 M, laquelle préparation contient de préférence un composé de formule I dans laquelle A21 est différent de Gln.

**8.** Préparation selon la revendication 5, caractérisée en ce que le composé est l'arginine ou la lysine.

**9.** Préparation selon les revendications 3, 4, 6, 7 ou 8 caractérisée en ce qu'elle contient des ions de zinc et/ou une protamine.

**10.** Préparation selon l'une quelconque des revendications 3 à 9, caractérisée en ce qu'elle possède une valeur de pH dans la plage d'environ 2,0 jusqu'à 8, de préférence de 2,5 jusqu'à 8.

**11.** Préparation selon l'une quelconque des revendications 3 à 9, caractérisée en ce qu'elle possède une valeur de pH dans la plage allant de 2,5 jusqu'à 4,5 ou de 6,5 jusqu'à 8,0.

**12.** Procédé pour la préparation de dérivés de l'insuline selon la revendication 1, dans lequel on met en réaction un composé précurseur biosynthétique ayant la formule générale II

INSUL-A21
|
X
(II)

dans laquelle A21 est tel que défini dans la revendication 1, et X est un liant, un reste d'acide aminé ou un reste de peptide reliant le groupe carboxyle de $Lys^{B29}$ au groupe amino de $Gly^{A1}$, avec un composé aminé de la formule générale III

Z-OR (III)

dans laquelle Z est Thr, Ala ou Ser où tout groupe hydroxy peut être protégé et R est un groupe protecteur carboxy utilisant une enzyme trypsine ou analogue à la trypsine en tant que catalyseur dans un mélange d'eau et de solvants organiques, après quoi on enlève le groupe protecteur carboxy et tout groupe protecteur hydroxy.

**13.** Procédé selon la revendication 12, dans lequel X est une partie de la formule IV

$-(Q_q-K)_r-$ (IV)

dans laquelle Q est une chaîne peptide avec des acides aminés q, q est un nombre entier de 0 à 33, K est Lys ou Arg, et r est égal à zéro ou à un.

**Revendications pour les Etats contractants suivants : ES, GR**

**1.** Procédé pour la préparation des dérivés de l'insuline de la formule générale I

INSUL-A21
|
B30
(I)

dans laquelle INSUL représente l'insuline humaine des(A21), des(B30) et A21 représente l'un des acides aminés Ala, Gln, Glu, Gly, His, Ile, Leu, Met, Phe, Ser, Thr, Trp, Tyr, Val ou hSer relié à $Cys^{A20}$ dans INSUL et B30 représente l'hydrogène ou l'un des acides aminés Ser, Ala ou Thr relié à $Lys^{B29}$

dans INSUL, et de préférence A21 est différent de Phe, dans laquelle on met en réaction un composé de précurseur biosynthétique ayant la formule générale II

$$\begin{array}{c} \text{INSUL-A21} \\ | \\ \text{X} \end{array} \qquad (II)$$

où A21 est tel que défini ci-dessus, et X est un liant, un reste d'acide aminé ou un reste peptide reliant le groupe carboxyle de $Lys^{B29}$ au groupe amino de $Gly^{A1}$, avec un composé aminé de la formule générale III

Z-OR (III)

dans laquelle Z est Thr, Ala ou Ser où tout groupe hydroxy peut être protégé et R est un groupe protecteur carboxy, utilisant une enzyme trypsine ou analogue à la trypsine en tant que catalyseur dans un mélange d'eau et de solvants organiques, après quoi on enlève le groupe protecteur carboxy et tout groupe protecteur hydroxy.

2. Procédé selon la revendication 1, dans lequel X est une partie de la formule IV

$-(Q_q\text{-}K)_r-$ (IV)

dans laquelle Q est une chaîne peptide avec des acides aminés q, q est un nombre entier de 0 à 33, K est Lys ou Arg, et r est égal à zéro ou à un.

3. Procédé selon la revendication 1 ou 2, dans lequel A21 représente Gly, Ala, Ser, Thr ou hSer et B30 représente Ala ou Thr.

4. Utilisation d'un dérivé de l'insuline selon la formule générale I

$$\begin{array}{c} \text{INSUL-A21} \\ | \\ \text{B30} \end{array} \qquad (I)$$

dans laquelle INSUL représente l'insuline humaine des(A21), des(B30), et A21 représente l'un des acides aminés Ala, Gln, Glu, Gly, His, Ile, Leu, Met, Phe, Ser, Thr, Trp, Tyr, Val ou hSer relié à $Cys^{A20}$ dans INSUL et B30 représente l'hydrogène ou l'un des acides aminés Ser, Ala ou Thr relié à $Lys^{B29}$ dans INSUL, et de préférence A21 est différent de Phe dans un procédé de fabrication d'une préparation pour le traitement du diabète sucré.

5. Utilisation selon la revendication 4, dans laquelle la préparation à fabriquer est soluble.

6. Utilisation selon la revendication 5, dans laquelle la préparation à fabriquer contient un composé qui facilite l'absorption.

7. Utilisation selon la revendication 6, dans laquelle le composé est un sel de magnésium.

8. Utilisation selon la revendication 7, dans laquelle la préparation à fabriquer est une solution ayant une valeur de pH dans la plage de 3-4 et contient des ions magnésium dans une concentration de 0,005 M à 0,5 M, laquelle préparation contient de préférence un composé de formule I où A21 est différent de Gln.

9. Utilisation selon la revendication 6, dans laquelle le composé est l'arginine ou la lysine.

10. Utilisation selon les revendications 4, 5, 7, 8 ou 9 dans laquelle la préparation à fabriquer contient des ions de zinc et/ou la protamine.

11. Utilisation selon l'une quelconque des revendications 4 à 10, dans laquelle la préparation à fabriquer possède une valeur de pH dans la plage allant de 2,0 jusqu'à 8, de préférence de 2,5 jusqu'à 8.

12. Utilisation selon l'une quelconque des revendications 4 à 10, dans laquelle la préparation à fabriquer possède une valeur de pH dans la plage allant de 2,5 jusqu'à 4,5 ou de 6,5 jusqu'à 8,0.